# EUROPÄISCHE PATENTSCHRIFT

(11) **EP 0 651 736 B1**
(45) Veröffentlichungstag und Bekanntmachung des Hinweises auf die Patenterteilung: **14.01.1998**
(21) Anmeldenummer: 93915975.2
(22) Anmeldetag: 22.07.1993
(51) Int. Cl.: C07C 229/34, C07C 229/06, C07C 229/30, C07C 227/32, C07D 207/26, C07D 207/28

(54) **VERFAHREN ZUR HERSTELLUNG ENANTIOMERENREINER beta-SUBSTITUIERTER gamma-AMINOBUTTERSÄUREDERIVATE, NEUE ENANTIOMERENREINE ZWISCHENSTUFEN DIESES VERFAHRENS UND DEREN VERWENDUNG**
PROCESS FOR PREPARING ENANTIOMER-PURE beta-SUBSTITUTED gamma-AMINOBUTYRIC ACID DERIVATES, NEW ENANTIOMER-PURE INTERMEDIATE STAGES OF SAID PROCESS AND THEIR USE
PROCEDE DE PREPARATION DE DERIVES D'ACIDE gamma-AMINOBUTYRIQUE beta-SUBSTITUES A FORME ENANTIOMERE PURE, NOUVELLES ETAPES INTERMEDIAIRES DE CE PROCEDE A FORME ENANTIOMERE PURE ET LEUR UTILISATION

(30) Priorität: 23.07.1992 DE 4224342
(43) Veröffentlichungstag der Anmeldung: 10.05.1995
(73) Patentinhaber: HERDEIS, Claus, 80809 München (DE)
(72) Erfinder: HERDEIS, Claus, D-80809 München (DE); HUBMANN, Hans-Peter, D-95326 Kulmbach (DE)
(74) Vertreter: Andrae, Steffen, Dr.
(86) Internationale Anmeldenummer: EP9301949
(87) Internationale Veröffentlichungsnummer: WO9402443

(56) Entgegenhaltungen:
- Tetrahedron: Asymmetri, Band 3, Nr. 9, 1992, C. HERDEIS et al.: "Synthesis of homochiral R-baclofen from S-glutamic acid 61", Seiten 1213-1221
- Tetrahedron Letters, Band 26, Nr. 49, 1985, E.J. COREY et al.: "Evidence for a reversible d,pi*-complexation, beta-cupration sequence in the conjugate addition reaction of Gilman reagents with alpha,beta-enones", Seiten 6015-6018
- Tetrahedrons Letters, Band 26, Nr. 5, 1985, HIDEO NAGASHIMA et al.: "Conjugate addition of organocopper reagents to N-tosylated alpha, beta-unsaturated amides", Seiten 657-660
- Synlett, Januar 1990, T.J. HAGEN: "Regio-selective functionalization of medium- ring lactams", Seiten 63-66
- J. Org. Chem., Band 46, 1981, H.J. CARLSEN et al.: "A greatly improved procedure for ruthenium tetraoxide catalyzed oxidation of organic compounds", Seiten 3936-3938
- Synthesis, November 1991, R.E. ZELLE: "Resolution of 4-Pheny l-2-pyrrolidinone: a versatile synthetic intermediate", Seiten 1023-1026
- J. Org. Chem., Band 48, 1983, D.L. FLYNN et al.: "A mild two-step method for the hydrolysis/methanolysis of secondary amides and lactams", Seiten 2424-2426
- Chemical Abstracts, Band 82, Nr. 23, 9. Juni 1975 (Columbus, Ohio, US), siehe Seite 526, Zusammenfassung Nr. 155373r, & JP,A,7440460 (TAKAHASHI, HIDEHIKO et al.) 2. November 1974

## Beschreibung

Die vorliegende Erfindung betrifft ein Verfahren zur Herstellung enantiomerenreiner β-substituierter γ-Aminobuttersäurederivate, insbesondere von R-Baclofen, neue enantiomerenreine Zwischenstufen dieses Verfahrens und deren Verwendung zur Herstellung von enantiomerenreinen substituierten Prolin- und Glutaminsäurederivaten.

Es besteht ein großes Interesse an wirksamen Verfahren, nach denen in Form optischer Isomerer vorkommende biologisch aktive Verbindungen ausgehend von leicht und kostengünstig verfügbaren Ausgangsverbindungen auf klassischem chemischem Wege in enantiomerenreiner Form erhalten werden können, da sich die biologischen Aktivitäten verschiedener Enantiomerer einer Verbindung in der Regel deutlich unterscheiden und die ausschließliche Verwendung des wirksamen Enantiomeren als Wirkstoff von z.B. Arzneimitteln häufig zu bevorzugen ist.

Zu den biologisch aktiven Verbindungen, die in Form verschiedener Enantiomerer mit unterschiedlicher Wirksamkeit vorkommen, gehören substituierte Aminosäuren wie z.B. die γ-Aminobuttersäuren, in deren Reihe sich die als Arzneimittelwirkstoff verwendete Verbindung Baclofen findet.

R-Baclofen ist ein Derivat des inhibitorischen Neurotransmitters GABA (γ-Aminobuttersäure). Es ist die lipophilste Substanz in dieser Klasse von Verbindungen mit hoher Affinität zum GABA_{B}-Rezeptor (IC₅₀ = 0.13µM) (W. Ziegelgänsberger und M. Hausser, GABA: Basic Research and Clinical Applications, Ed. N. G. Bowery and G. Nisticò, p. 87, Pythagora Press Rome-Milan (1989); N. G. Bowery, D. R. Hill, A. L. Hudson, A. Doble, D. N. Middlemiss, J. Shaw und M. Turnbull, Nature, 1980, **283**, 92-94; N. G. Bowery, D. R. Hill und A. L. Hudson, Br. J. Pharmacol. 1983, **78**, 191-206; R. Chenevert und M. Desjardins, Tetrahedron Lett. 1991, 32, 4249-4250; H. R. Olpe, H. Demiéville, V. Baltzer, W. L. Bencze, W. P. Koella, P. Wolf und H. L. Haas, Eur. J. Pharm. 1978, 52, 133-136). Die racemische Form, RS-Baclofen (Lioresal^{R}), wird zur Behandlung von Spasmen benutzt, die bei Erkrankungen des Rückenmarks, speziell nach traumatischen Verletzungen, auftreten können. Da S-Baclofen eine sehr geringe Affinität zum GABA_{B}-Rezeptor besitzt, ist es wünschenswert, R-Baclofen in enantiomerenreiner Form einzusetzen bzw. eine Synthese des R-Baclofens zu konzipieren. Kürzlich wurde eine chemoenzymatische Synthese des R- and S-Baclofens beschrieben (R. Chenevert und M. Desjardins, Tetrahedron Lett. 1991, 32, 4249-4250).

Es ist Aufgabe der vorliegenden Erfindung, ein Verfahren zu schaffen, das die Synthese von enantiomerenreinen substituierten Aminosäurederivaten, insbesondere auch von β-substituierten γ-Aminobuttersäurederivaten einschließlich R-Baclofen, ausgehend von leicht verfügbaren Ausgangssubstanzen auf chemischem Wege in guten Ausbeuten und hoher Reinheit ermöglicht.

Diese Aufgabe wird gelöst durch ein Verfahren gemäß Anspruch 1, die im Rahmen eines derartigen Verfahrens zugänglichen enantiomerenreinen Zwischenstufen gemäß den Ansprüchen 10 und 11 sowie deren Verwendungen.

Das erfindungsgemäße Verfahren wurde im Rahmen unserer Arbeiten zur Synthese nichtproteinogener Aminosäuren (C. Herdeis und W. Engel, Tetrahedron: Asymmetry, 1991, 2, 945-948) geschaffen. Ausgehend von unserer Erkenntnis, daß aus natürlicher L-Glutaminsäure auf einfachem Wege enantiomerenreine S-Pyroglutaminsäurederivate erhältlich sind und diese dann, wenn es gelingt, in derartige S-Pyroglutaminsäurederivate stereoselektiv Substituenten in die 4-Position einzuführen, in enantiomerenreine γ-Aminobuttersäurederivate überführt werden können, untersuchten wir die 1,4-konjugierte Addition metallorganischer Reagenzien an die stark elektrophile Doppelbindung des bekannten S-Pyroglutaminsäurederivats (IIa). Die Herstellung von (IIa) ist beschrieben in: K. Jones und K.-C. Woo, Tetrahedron Lett. 1991, 32, 6949-6952. In dieser Verbindung (IIa) aktiviert die Acceptoreigenschaft der Boc-Gruppe (tert.-Butoxycarbonylgruppe) am Amidstickstoff das α,β-ungesättigte System gegenüber der konjugierten Addition von Gilman-Cupraten im gewünschten Sinne (vgl. zur Grundreaktion: T. J. Hagen, Synlett. 1990, 63-66; P. Somfai, H. Ming He und D. Tanner, Tetrahedron Lett. 1991, 32, 283-286; K. Itoh, H. Nagashima, N. Ozaki und M. Washiyama, Tetrahedron Lett. 1985, 26, 657-660). Die tert.-Butyldiphenylsilylschutzgruppe wurde von uns aufgrund ihres sterischen Raumbedarfs gewählt, da wir hofften, daß dadurch die von uns gewünschte trans-Addition in der 4-Position des Rings selektiv bevorzugt würde. Diese Hoffnung bestätigte sich. Obwohl alle in der vorliegenden Anmeldung beschriebenen konkreten Reaktionen mit der Schutzgruppe der Verbindung (IIa) durchgeführt wurden, ist zu erwarten, daß der Fachmann in Kenntnis der in der vorliegenden Anmeldung offenbarten Reaktionssequenz ohne größere Probleme andere sterisch anspruchsvolle Schutzgruppen finden kann, die ebenfalls eine selektive trans-Addition im Sinne der vorliegenden Erfindung fördern. Beispiele für derartige Schutzgruppen sind die tert.-Butyldimethylsilylgruppe und die Tritylgruppe.

Wir fanden ferner, daß sich die Anwendungsbreite der Verbindung (IIa) beträchtlich erweitern ließ, als wir außer Gilman-Cupraten andere metallorganische Verbindungen zur der 1,4-Addition erfolgreich einsetzten.

So untersuchten wir die Addition anderer Organocuprate, z.B. Grignardcuprate, mit der Absicht, das synthetische Potential des Pyroglutaminsäurederivats (IIa) auszuloten und einen möglichen Syntheseweg zum R-Baclofen zu eröffnen.

Die bekannte Herstellung von (IIa) erfolgt ausgehend von L-Glutaminsäure in sechs Schritten:

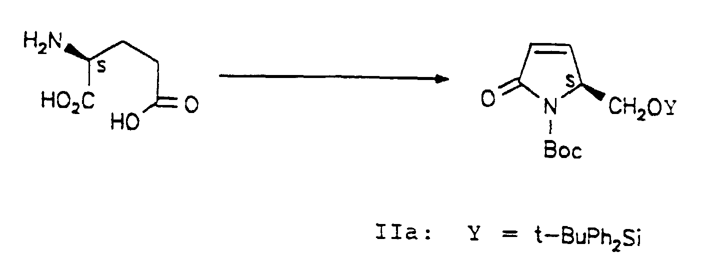

Es stellte sich heraus, daß die Reaktion von (IIa) mit fünf Äquivalenten der Gilmancuprate (R=CH₃, n-Bu, Ph) in Anwesenheit von Trimethylsilylchlorid (TMSCl) (E. Nakamura, S. Matsuzawa, Y. Horiguchi und I. Kuwajima, Tetrahedron Lett. 1986, 27, 4029-4032; C. Scolastico, S. Cardani, G. Poli und R. Villa, Tetrahedron, 1988, **44**, 5929-5938; E. J. Corey und N. W. Boaz, Tetrahedron Lett. 1985, **26**, 6015-6018; A. Alexakis, J. Berlan, und J. Besace, Tetrahedron Lett. 1986, **27**, 1047-1050) die Verbindungen (IIIa-c) in ausgezeichneten Ausbeuten lieferte. Die Verbindungen (IIId-f) wurden durch Addition der Grignardcuprate (R=vinyl, p-Cl-Ph, allyl) in etwas niedrigerer, aber trotzdem zufriedenstellender Ausbeute erhalten (60-70%). Hierbei konnten die cis-isomeren Verbindungen weder durch ¹H- noch durch ¹³C-NMR Spektroskopie in den Rohprodukte der Reaktion nachgewiesen werden.

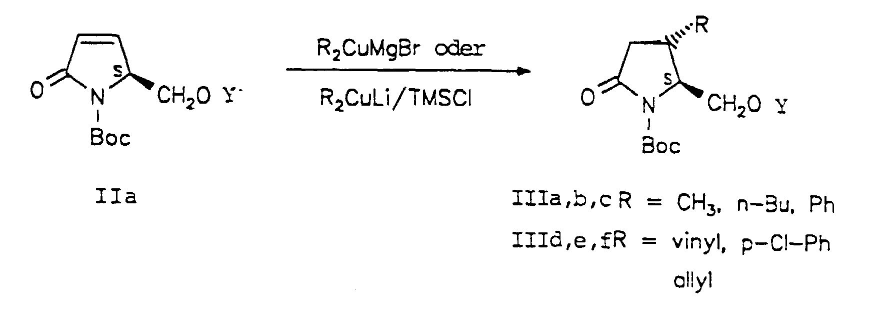

Es ist bemerkenswert, daß nur ein großer Überschuß der Gilman-bzw. Grignardcuprate (5 Äquivalente) zufriedenstellende Ausbeuten an Michael-Additionsprodukten lieferte. Dies stimmt mit Ergebnissen von Hanessian und Tamm (S. Hanessian und K. Sumi, Synthesis, 1991, 1083-1089; C. Tamm und M. Matthes, Helv. Chim. Acta, 1991, **74**, 1585-1590) und unseren eigenen Versuchen an Vinylsulfonen als Substrate (C. Herdeis und C. Hartke-Karger, Liebigs Ann. Chem. 1991, 99-104) überein. Entgegen der Tatsache, daß ein großer Überschuß an metallorganischem Reagenz den γ-Lactamring öffnen sollte (T. J. Hagen, Synlett. 1990, 63-66), konnte kein ringoffenes Produkt nachgewiesen werden. Dies könnte dadurch erklärt werden, daß das intermediär entstehende Amidenolat durch das Trimethylsilylchlorid abgefangen wird.

Die Abspaltung der Silylschutzgruppe und die damit erfolgende Freisetzung der OH-Funktion ohne Verlust der Boc-Gruppe erfolgte durch Triethylammoniumfluorid (S. Hünig und G. Wehner, Synthesis, 1975, 180-182) in THF. Trotz der langen Reaktionszeit (4 Tage) ist dieses Reagens hoch chemoselektiv. Die übliche Entfernung der Silylschutzgruppe durch Tetrabutylammoniumfluorid in THF verlief, bedingt durch die hohe Nucleophilie des Fluoridions, unter Ringöffnung. Die Oxidation der primären OH-Funktion zum Pyroglutaminsäurederivat (Ve), gelang in einem Schritt mit der Sharpless Methode (K. B. Sharpless, P. H. J. Carlsen, T. Katsuki und V. S. Martin, J. Org. Chem. 1981, **46**, 3936-3938). Dies erwies sich als sehr vorteilhaft, da so größere Mengen von Cr^{VI} (z.Bp. PDC/DMF) zur Oxidation von (IVe) vermieden werden konnten. (Ve) wurde durch Extraktion ohne Säulenchromatographie als kristallines Material erhalten.

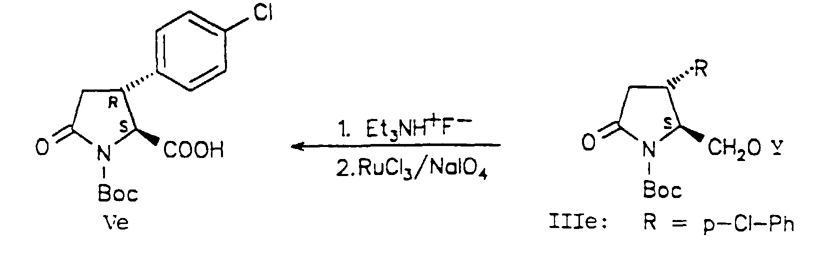

D. H. R. Barton und Mitarbeiter beschrieben eine Reaktionssequenz zur Decarboxylierung N-geschützter Aminosäuren und Peptide. Alle Chiralitätszentren, die nicht von der Radikalreaktion betroffen sind, bleiben als stereogene Zentren erhalten (D. H. R. Barton, Y. Hervé, P. Potier und J. Thierry, Tetrahedron, 1988, **44**, 5479-5486). Wir wendeten diese Reaktion auf (Ve) an, und erhielten unter Decarboxylierung das γ-Aminobuttersäurederivat (VIe) in 60-65% Ausbeute.

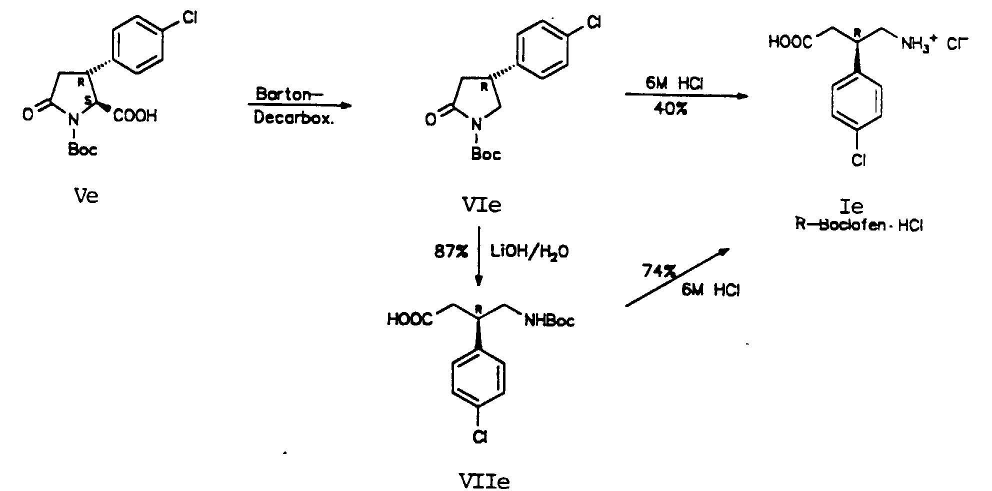

Dieses cyclische Boc-Derivat des R-Baclofens, (VIe), wurde auf zwei Wegen in R-Baclofen-Hydrochlorid umgewandelt. Durch Ringöffnung von (VIe) nach der von Grieco beschriebenen Methodik mit 1M LiOH/THF (P. A. Grieco, D. L. Flynn, und R. E. Zelle, J. Org. Chem. 1983, **48**, 2424-2426; R. E. Zelle, Synthesis, 1991, 1023-1026) erhielt man in 87% Ausbeute (VIIe), das durch Behandlung mit 6M Salzsäure in 74% Ausbeute in R-Baclofen^{·}HCl (Ie) umgewandelt wurde. Alternativ lieferte die Hydrolyse des Lactams (VIe) R-Baclofen^{·}HCI (Ie) nur in 40% Ausbeute. Die Enantiomerenreinheit des auf diesen zwei Wegen erhaltenen R-Baclofen^{·}HCl (Ie) ist in Übereinstimmung mit Material, das durch Racemattrennung (H. R. Olpe, H. Demiéville, V. Baltzer, W. L. Bencze, W. P. Koella, P. Wolf und H. L. Haas, Eur. J. Pharm. 1978, **52**, 133-1362) und chemoenzymatische Synthese (R. Chenevert und M. Desjardins, Tetrahedron Lett. 1991, 32, 4249-4250) erhalten wurde.

Zusammenfassend kann gesagt werden, daß diese 5stufige Reaktionssequenz (ausgehend von IIa) einen einfachen Zugang zu R-Baclofen in 16% Gesamtausbeute bietet. Darüberhinaus ist L-Glutaminsäure bzw. L-Pyroglutaminsäure ein wohlfeiles Ausgangsmaterial aus dem chiralen pool der natürlichen Aminosäuren.

Angesichts der durch die nachfolgenden Beispiele belegten Tatsache, daß die verschiedensten Reste R stereoselektiv in die 4-Position des Pyroglutaminsäurerings eingeführt werden können, ist es für den Fachmann klar ersichtlich, daß die Reaktion nicht nur zur Herstellung von R-Baclofen genutzt werden kann, sondern nahezu beliebig substituierte γ-Aminobuttersäuren zugänglich macht. Die während des erfindungsgemäßen Verfahrens durchlaufenen Zwischenstufen sind - mindestens in enantiomerenreiner Form - neue Verbindungen, die nicht nur im Sinne des erfindungsgemäßen Verfahrens verwendet werden können. So kann eine Verbindung der allgemeinen Formel (III) zur Herstellung enantiomerenreiner substituierter Proline verwendet werden, indem man in geeigneter Reihenfolge die Ringcarbonylgruppe reduziert, die Methylolseitenkette zur Carboxylgruppe oxidiert und die Boc-Gruppe abspaltet. In ähnlicher Weise können Verbindungen der Formel (V) zur Herstellung enantiomerenreiner substituierter Glutaminsäurederivate verwendet werden, indem man statt zu decarboxylieren den Ring öffnet und die Boc-Gruppe abspaltet.

Eine weitere als Arzneimittelwirkstoff interessante Verbindung, die nach dem erfindungsgemäßen Verfahren in enantiomerenreiner Form synthetisiert werden kann, ist die Verbindung 4-(3-Cyclopentyloxy-4-methoxy-phenyl)-2-pyrrolidon (Rolipram), die zu den enantiomerenreinen in 4-Position substituierten 2-Prryolidonen gehört, die man erhält, indem man als Rest R einen 3-Cyclopentyloxy-4-methoxyphenylrest in das S-Pyroglutaminsäuremolekül einführt und nach der Decarboxylierung der Verbindung (V) aus der erhaltenen Verbindung (VI) die Boc-Schutzgruppe abspaltet.

Nachfolgend soll die Erfindung unter besonderer Berücksichtigung der Herstellung von R-Baclofen anhand von Ausführungsbeispielen noch näher erläutert werden.

**Experimenteller Teil**
**Allgemeines**: THF wurde vor dem Gebrauch über Lithiumaluminiumhydrid destilliert, anschließend über Na-Draht unter Stickstoff. Organometall-Reaktionen wurden in ausgeflammtem Kolben unter trockenem und O₂ freiem Stickstoff durchgeführt. Dünnschichtchromatographie wurde auf Merck Silicagel Platten 60 F-254 durchgeführt. ¹H-NMR und ¹³C-NMR Spektren wurden auf einem Bruker AC 200 Gerät gemessen. Abkürzungen: FM: Fließmittel für DC, PE: Petrolether, EtOAc. Ethylacetat, THF: Tetrahydrofuran, Boc: tert.-Butoxycarbonyl, TMSCl: Trimethylsilylchlorid, SC: Säulenchromatographie.

### Beispiel 1: Herstellung der Ausgangsverbindung (IIa)

### 5S-1-tert.Butoxycarbonyl-5-tert.butyldiphenylsilyloxymethyl-1,5-dihydro-2H-pyrrol-2-on (IIa)

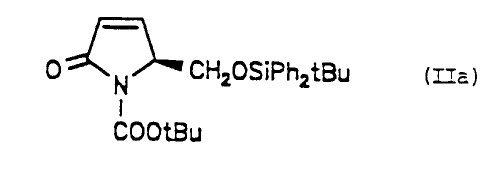

7,3 ml (35 mmol) Hexamethyldisilazan werden in einem ausgeheiztem Schlenk unter N₂ in 50 ml THF gelöst, auf -78°C abgekühlt und mit 17,5 ml (35 mmol) Butyllithium (BuLi) 2M versetzt. Nach 5 min Rühren bei dieser Temperatur läßt man noch 30 min bei 0°C rühren, kühlt wieder auf -78°C und gibt langsam 6,8 g (15mmol) 5S-1-tert.Butoxycarbonyl-5--tert.butyldiphenylsilyloxymethyl-pyrrolidin-2-on in 30 ml THF zu. Nach 30 min Rühren bei -78°C werden 3,54 g (18,5 mmol) PhSeCl in 20 ml THF zugegeben und weitere 2 h bei dieser Temperatur gerührt. Der Ansatz wird mit gesättigter NH₄Cl-Lösung abgestoppt, mit Et₂O verdünnt und die organische Phase solange mit gesättigter NH₄Cl-Lösung gewaschen, bis in der Wasserphase sich kaum mehr Niederschlag bildet. Nach Waschen mit gesättigter NaCl-Lösung und Trocknen über Na₂SO₄ wird das Lösungsmittel im Vakuum abgezogen. Es verbleibt ein oranges, dickflüssiges Öl, das noch etwas Edukt enthält. Dieses und andere, polare Verunreinigungen werden durch Säulenchromatographie (SC) mit PE/EtOAc 2+1 abgetrennt. Das eingeengte Eluat wird in 50 ml EtOAc gelöst und mit 15 ml 30% H₂O₂-Lösung versetzt. Nach ca. 45 min ist die Lösung nur noch schwach gelb gefärbt bis farblos und wird nach Verdünnen mit EtOAc mit gesättigter NaHCO₃-Lösung solange gewaschen, bis die Wasserphase nicht mehr rot gefärbt ist. Es wird noch einmal mit gesättigter NaCl-Lösung gewaschen, über Na₂SO₄ getrocknet und im Vakuum eingeengt. Es verbleibt ein gelbliches Öl, das mit Diisopropylether versetzt und aufgekocht wird. In der Hitze wird ungefähr das gleiche Volumen Petrolether zugesetzt und die Kristallisation durch Reiben mit einem Glasstab eingeleitet. Es fallen 4,4 g fast farblose Kristalle aus, die Mutterlauge wird nochmals gleichartig behandelt und liefert noch 0,45 g Kristalle gleichen Schmelzpunktes.

Ausbeute: 4,85 g (71%) schwach gelbliche Kristalle (R_{f} = 0,56 FM: PE/EtOAc 2+1)
Schmp.: 96°C (Diisopropylether/PE)
IR (KBr): 2900, 1725 (C=O Urethan), 1690 (C=O Lactam), 1450, 1420, 1360, 1340, 1310, 1145, 1110, 910, 795, 730, 700 cm⁻¹
¹H-NMR (CDCl₃): d (ppm) =1.03 (9H, s, t-Bu-Si) 1.44 (9 H, s, t-Bu-O), 3.83 (1 H, dd, J_{gem}=9,7 Hz, J₅₋₆ₐ=6,4 Hz, H-6), 4.12 (1 H, dd, J_{gem}=9,7 Hz, J_{5-6b} =3, 5 Hz, H-6), 4.64 (1 H, m, H-5), 6.17 (1 H, dd, J₃₋₄= 6,13 Hz, J₃₋₅=1,5 Hz, H-3), 7.26 (1 H, dd, J₃₋₄=6,1 Hz, J₄₋₅=2,05 Hz, H-4), 7.38 (6 H, m, H-Ar), 7.60 (4 H, m, H-Ar)
¹³C-NMR (CDCl₃): d (ppm) = 19.2 (SiC(CH₃)₃), 26.67 (SiC(CH₃)₃), 27.97 (OC(CH₃)₃), 62.93 (C-5), 63.42 (C-6), 82.65 (OC(CH₃)₃), 127.32 (C-3), 127.79 (Ar), 129.69 (C-4), 132.64 (Ar), 132.86 (Ar), 135.43 (Ar), 149.29 (C=O Urethan), 169.48 (C-2)
[α]_{D}²⁰ = -124 (c=0, 23 /CHCl₃)

| | | | | | | |
|---|---|---|---|---|---|---|
| C₂₆H₃₃NO₄Si 451,64 | | | | | | |
| ber. | C | 69,14% | H | 7,36% | N | 3,10% |
| gef. | C | 69,61% | H | 7,70% | N | 3,09% |

### Beispiel 2: Herstellung von γ-Aminobuttersäureverbindungen

### Stufe 1-A: 4S,5S-1-tert.-Butoxycarbonyl-5-tert.butyl-diphenylsilyloxymethyl-4-methylpyrrolidin-2-on (IIIa)

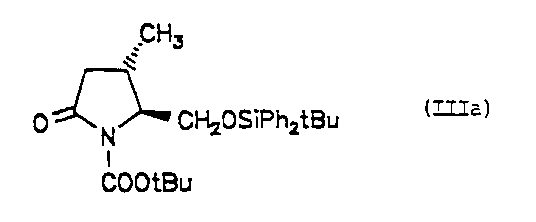

In einem ausgeheizten Schlenk werden unter N₂ 0,52 g (2,5 mmol) CuBrSMe₂ in 5 ml Et₂O suspendiert und bei -20°C 3,12 ml (5mmol) Methyllithium 1,6 M in Et₂O zugegeben. Nach 15 min Rühren bei dieser Temperatur kühlt man auf -78°C und gibt 225 mg (0,5 mmol) IIa mit 0,1 ml (lmmol) TMSCl in 3 ml THF zu. Der Ansatz wird 1 h bei -78°C gerührt, mit gesättigter NH₄Cl-Lösung abgestoppt und mit 10 ml EtAc verdünnt. Es wird solange mit NH₄Cl-Lösung gewaschen, bis die wässrige Phase keine Blaufärbung mehr zeigt. Man wäscht noch einmal mit NaCl-Lösung, trocknet über Na₂SO₄ und destilliert das Lösungsmittel am Rotavapor ab. Das zurückbleibende, schwach gelbliche Öl wird durch SC an SiO₂ mit Et₂O/PE 2+3 gereinigt. Nach Einengen verbleibt ein farbloses Öl, das durch Anreiben mit n-Hexan zur Kristallisation gebracht wird. Nach Umkristallisation aus EtOAc/ n-Hexan erhält man weiße Kristalle.
Ausbeute: 190 mg (81%) weiße Kristalle (R_{f}=0,3 FM: Et₂O/PE 2+3)
Smp: 85°C (EtOAC/n-Hexan)
IR (KBr): 3080, 2950, 2860, 1790, 1750, 1715, 1590, 1470, 1430, 1370, 1310, 1160, 1110, 780, 700 cm⁻¹
¹H-NMR (CDCl₃): d (ppm) = 1.04 (9 H, s, t-Bu-Si), 1.14 (3 H, d, J=7,1 Hz, CH3), 1.43 (9 H, s, t-Bu-O), 2.07 (1 H, dd, J₃ₐ₋₄=1,8 Hz, J_{gem}=17,5 Hz, H-3), 2.44 (1 H, quin, J=7,2 Hz, H-4), 2.98 (1 H, dd, J_{3b-4}=8,6 Hz, J_{gem}= 17,5 Hz, H-3), 3.72 (2 H, m, H-5 und H-6), 3.86 (1 H, m, H-6), 7.39 (6 H, m, H-Ar), 7.61 (4 H, m, H-Ar),
¹³C-NMR (CDCl₃): d (ppm) = 19.2 (SiC(CH₃)₃), 21.44 (CH₃), 28.81 (SiC(CH₃)₃), 28.02 (OC(CH₃)₃), 28.26 (C-4), 40.47 (C-3), 64.23 (C-5), 66.21 (C-6), 82.72 (OC(CH₃)₃), 127.85 (C-Ar), 129.89 (C-Ar), 135.11 (C-Ar), 135.56 (C-Ar), 150.03 (Urethan), 174.39 (Lactam)
[α]_{D}²⁰ = -33 (c=0,4/CHCl₃)

| | | | | | | |
|---|---|---|---|---|---|---|
| C₂₇H₃₇NO₄Si 467,68 | | | | | | |
| ber. | C | 69,34% | H | 7,97% | N | 2,99% |
| gef. | C | 69,28% | H | 7,93% | N | 3,00% |

### Stufe 1-B: 4S,5S-1-tert.-Butoxycarbonyl-4-butyl-5-tert.-butyldiphenylsilyloxymethylpyrrolidin-2-on (IIIb)

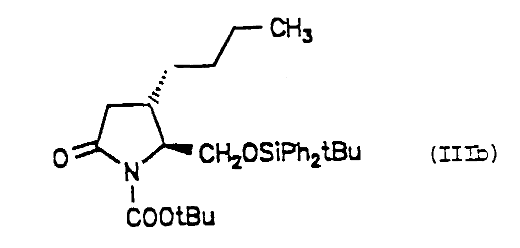

In einem ausgeheizten Schlenk werden 1,04 (5mmol) CuBrSMe₂ in 7 ml Et₂O suspendiert und bei -15°C mit 6,25 ml (10 mmol) BuLi 1,6 M in Hexan versetzt. Nach 15 min Rühren wird auf -78°C gekühlt und 450 mg (1 mmol) IIa mit 0,2 ml (2 mmol) TMSCl in 5 ml THF zugegeben. Der Ansatz wird 1 h gerührt, mit gesättigter NH₄Cl-Lösung abgestoppt und wie bei IIIa beschrieben aufgearbeitet. Man erhält 484 mg eines farblosen Öles.
Ausbeute: 484 mg (95%) farbloses Öl (R_{f} = 0,5 FM: Et₂O/PE 2+3)
IR (Film): 3080, 2950, 2860, 1790, 1750, 1710, 1590, 1470, 1430, 1370, 1310, 1150, 1110, 790, 700 cm⁻¹
¹H-NMR (CDCl₃): d (ppm) = 0.90 (3 H, t, J=6,5 Hz, CH₃-Bu), 1.04 (9 H, s, t-Bu-Si), 1.29 (6 H, m, CH₂-Bu), 1.44 (9 H, s, t-Bu-O), 2.16 (1 H, dd, J₃ₐ₋₄= 1,8 Hz, J_{gem}=17,5 Hz, H-3), 2.26 (1 H, m, H-4), 2.91 (1 H, dd, J_{3b-4}= 8,6 Hz, J_{gem}=17,6 Hz, H-3), 3.69 (1 H, dd, J₆ₐ₋₅=4,4 Hz, J_{gem}= 12,1 Hz, H-6), 3.83 (2 H, m, H-5 und H-6), 7.39 (6 H, m, H-Ar), 7.62 (4 H, m, H-Ar)
¹³C-NMR (CDCl₃): d (ppm) = 13.96 (CH₃-nBu), 19.18 (SiC(CH₃)₃), 22.51 ( CH₂-nBu), 26.76 (SiC(CH₃)₃), 27.99 (OC(CH₃)₃), 29.00 (C-4), 33.28 (CH₂-nBu), 35.04 (CH₂-nBu), 38.66 (C-3), 64.48 (C-5), 64.54 (C-6), 82.74 (OC(CH₃)₃), 127.80 (C-Ar), 129.85 (C-Ar), 133.07 (C-Ar), 135.50 (C-Ar), 150.04 (Urethan), 174.55 (Lactam) [α]_{D}²⁰ = -31 (c=O,22/CHCl₃)

| | | | | | | |
|---|---|---|---|---|---|---|
| C₃₀H₄₃NO₄Si 509,76 | | | | | | |
| ber. | C | 70,69% | H | 8,50% | N | 2,75% |
| gef. | C | 70,63% | H | 8,74% | N | 2,71% |

### Stufe 1-C: 4R,5S-1-tert.-Butoxycarbonyl-5-tert.butyldiphenylsilyloxymethyl-4-phenylpyrro-lidin-2-on (IIIc)

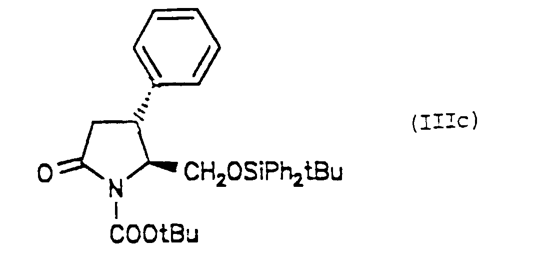

In einem ausgeheizten Schlenk werden 0,52 g (2,5 mmol) CuBrSMe₂ in 5 ml Et₂O suspendiert und bei -35°C mit 2,5 ml (5 mmol) PhLi 2 M versetzt. Nach 15 min Rühren kühlt man auf -78°C und gibt 225 mg (0,5 mmol) IIa mit 0,1 ml (lmmol) TMSCl in 3 ml THF zu. Der Ansatz wird 1,5 h gerührt und mit gesättigter NH₄Cl-Lösung abgestoppt. Die Aufarbeitung erfolgt wie unter IIIe beschrieben. Man erhält ein farbloses, zähes Harz.
Ausbeute: 160 mg (60%) farbloses Harz, (R_{f} = 0,44 FM: Et₂O/PE 2+3)
IR (Film): 3080, 2950, 2860, 1790, 1750, 1715, 1610, 1590, 1430, 1370, 1310, 1150, 1110, 790, 700 cm⁻¹
¹H-NMR (CDCl₃): d (ppm) = 1.09 (9 H, s, Si-t-Bu), 1.42 (9 H, s, O-t-Bu), 2.57 (1 H, dd, J₃ₐ₋₄=2,63 Hz, J_{gem}=17,8 Hz, H-3), 3.21 (1 H, dd, J_{3b-4}=9,6 Hz, J_{gem}=17,8 Hz, H-3), 3.50 (1 H, dt, J₃ₐ₋₄J₄₋₅=2,3 Hz, J_{3b-4}=9,5 Hz, H-4), 3.81 (1 H, dd, J₆ₐ₋₅=2,3 Hz, J_{gem}=10,5 Hz, H-6), 3.98 (1 H, dd, J_{6b-5}= 4,25 Hz, J_{gem}=10,5 Hz, H-6), 4.12 (1 H, m, H-5), 7.32 (11 H, m, H-Ph und H-Ar-Si), 7.66 (4 H, m, H-Ar-Si)
¹³C-NMR (CDCl₃): d (ppm) = 19.22 (SiC(CH₃)₃), 26.85 (SiC(CH₃)₃), 27.95 (OC(CH₃)₃), 38.68 (C-4), 39.91 (C-3), 64.22 (C-5), 66.62 (C-6), 83.04 (OC(CH₃)₃), 126.39 (C-Ar), 127.13 (C-Ar), 127.88 (C-Ar), 129.06 (C-Ar), 129.95 (C-Ar), 132.57 (C-Ar), 132.93 (C-Ar), 135.55 (C-Ar), 144.03 (C-Ar), 149.62 (Urethan), 174.12 (Lactam) [α]_{D}²⁰ = -27 (c=0,23/CHCl₃)

| | | | | | | |
|---|---|---|---|---|---|---|
| C₃₂H₃₉NO₄Si 529,75 | | | | | | |
| ber. | C | 72,55% | H | 7,42% | N | 2,64% |
| gef. | C | 72,67% | H | 7,64% | N | 2,51% |

### Stufe 1-D: 4R,5S-1-tert.Butoxycarbonyl-5-tert.-butyldiphenylsilyloxymethyl-4-vinyl-pyrroli-din-2-on (IIId)

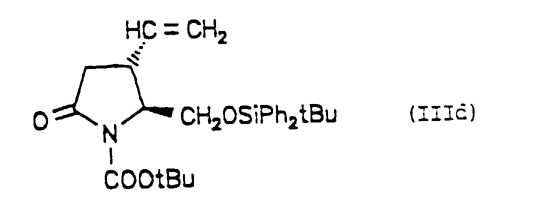

In einem ausgeheizten Schlenk werden unter N₂ 0,52 g (2,5 mmol) CuBrSMe₂ in 5 ml absolutem Et₂O suspendiert und bei - 30°C 5,0 ml (5 mmol) Vinylmagnesiumbromid (1 M in THF) zugespritzt. Nach 15 min Rühren kühlt man auf -78°C und tropft 225 mg (0,5 mmol) IIa mit 0,1 ml (1 mmol) TMSCl in 4 ml THF zu. Nach 1 h wird der Ansatz mit gesättigter NH₄Cl-Lösung abgestoppt und mit 15 ml EtOAc verdünnt. Es wird solange mit NH₄Cl-Lösung und NH₃ gewaschen, bis die Wasserphase nicht mehr blau gefärbt ist. An der Grenzfläche bleibt dabei ein rotbrauner Niederschlag bestehen. Nach Waschen mit NaCl-Lösung wird über Na₂SO₄ getrocknet und das Lösungsmittel am Rotavapor abdestilliert. Das zurückbleibende, gelbliche Öl wird mit SC an SiO₂ mit Et₂O/PE 2+3 gereinigt. Man erhält ein farbloses Öl. Ausbeute: 150 mg (62%) farbloses Öl (R_{f}=0,38 FM: Et₂O/PE 2+3)
IR (Film): 3070, 2930, 2850, 1790, 1750, 1710, 1590, 1470, 1425, 1360, 1310, 1150, 1110, 970, 920, 870, 780, 700 cm⁻¹ ¹H-NMR (CDCl₃): d (ppm) = 1.05 (9 H, s, tBuSi), 1.43 (9 H, s, tBuO), 2.33 (1 H, dd, J₃ₐ₋₄= 6,3 Hz, J_{gem}=20,9 Hz, H-3), 3.00 (2 H, m, J_{3b-4}=9 Hz, J_{gem}=21 Hz, H-3 und H-4), 3.74 (1 H, dd, J₅₋₆ₐ=3,65 Hz, J_{gem}=11,8 Hz, H-6), 3.93 (1 H, m, J_{5-6b}=4,1 Hz, J_{gem}=11,85 Hz, H-5 und H-6), 5.08 (2 H, m, =CH₂), 5.87 (1 H, ddd, Jₜᵣₐₙₛ=17,4 Hz, J_{cis}=10 Hz, J_{4-Vinyl}=7,4 Hz, -CH=), 7.41 (6 H, m, H-Ar), 7.63 (4 H, m, H-Ar)
¹³C-NMR (CDCl₃): d (ppm) =19.18 (SiC(CH₃)₃), 26.79 (SiC(CH₃)₃), 27.97 (OC(CH₃)₃), 37.03 (C-4), 37.92 (C-3), 63.93 (C-5), 64.35 (C-6), 82.88 (OC(CH₃)₃), 115.20 (=CH₂), 127.85 (C-Ar), 129.91 (C-Ar), 132.58 (C-Ar), 132.95 (C-Ar), 135.52 (C-Ar), 139.15
(-CH=), 149,74 (Urethan), 173.80 (Lactam)
[α]_{D}²⁰ = -23 (c=0,21/CHCl₃)

| | | | | | | |
|---|---|---|---|---|---|---|
| C₂₈H₃₇NO₄Si 479, 69 | | | | | | |
| ber. | C | 70,11% | H | 7,77% | N | 2,92% |
| gef. | C | 70,12% | H | 7,78% | N | 2,92% |

### Stufe 1-E: 4R,5S-1-tert.-Butoxycarbonyl-5-tert.-butyldiphenylsilyloxymethyl-4-(4-chlor-phenyl)pyrro lidin-2-on (IIIe)

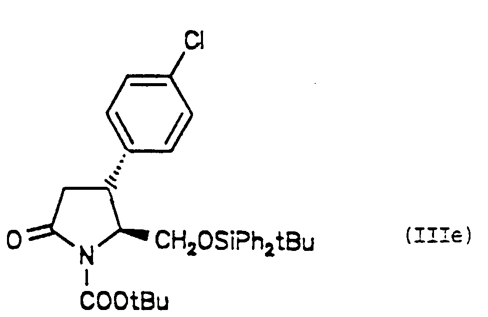

In einem ausgeheizten Schlenk werden unter N₂ 3,28 g (16mmol) CuBrSMe₂ in 10 ml absolutem Ether suspendiert und bei - 35°C 32 ml (32mmol) p-Chlorphenylmagnesiumbromid (1 M in Et₂O) zugegeben. Nach 20 min Rühren kühlt man auf -78°C und gibt 1,35 g (3mmol) IIa in 10 ml THF zusammen mit 0,6 ml (6mmol) TMSCl dazu. Sofort fällt ein rotbrauner Niederschlag aus und der Ansatz wird schwer rührbar. Man läßt langsam auf Raumtemperatur hochkommen, dabei wird die Suspension homogen. Nach 1 h wird mit halbgesättigter NH₄Cl-Lösung abgestoppt, der Ansatz mit Ether verdünnt und die Etherphase solange mit NH₄Cl-Lösung gewaschen, bis die Wasserphase nicht mehr blau gefärbt ist. Zum Schluß wird mit gesättigter NaCl-Lösung gewaschen, über Na₂SO₄ getrocknet und das Lösungsmittel abrotiert. Das im Rückstand verbliebene Chlorbenzol wird mit einer Mikrodestille entfernt und das zurückbleibende gelbe Öl durch SC mit Et₂O/PE 2+3 gereinigt. Das Rohprodukt wird in Ether aufgenommen und mit 0,5 N NaOH 3 mal gewaschen, um bei der Reaktion entstandenes p-Chlorphenol zu entfernen. Nach Trocknen und Abziehen des Ethers erhält man ein farbloses, zähes Öl. Ausbeute: 1,12 g (66%) farbloses Öl (R_{f} = 0,4 FM = Et₂O/PE 3+2)
IR (Film): 2930, 1790, 1750, 1710, 1500, 1430, 1370, 1310, 1150, 1110, 700 cm⁻¹
¹H-NMR (CDCl₃): d (ppm) = 1.09 (9 H, s, t-Bu-Si), 1.42 (9 H, s, t-Bu-O), 2.53 (1 H, dd, J_{gem}=17,8 Hz, J₃ₐ₋₄=2,5 Hz,
H-3), 3.20 (1 H, dd, J_{gem}=17,8 Hz, J_{3b-4}=9,5 Hz, H-3), 3.48 (1 H, dt, J_{3b-4}=9,5 Hz, J₃ₐ₋₄J₄₋₅=2,1 Hz, H-4), 3.80 (1 H, dd, J₅₋₆ₐ=2,3 Hz, J_{gem}=10,43 Hz, H-6), 3.97 (1 H, dd, J_{5-6b}=4,31 Hz, J_{gem}=10,43 Hz, H-6), 4.05 (1 H, m, H-5), 7.08 (2 H, dd, J_{AB}=8,4 Hz, H-Ar-p-Cl), 7.28 (2 H, dd, J_{AB}=8,4 Hz, H-Ar-p-Cl), 7.40 (6 H, m, H-Ar-Si), 7.64 (4 H, m, H-Ar-Si)
¹³C-NMR (CDCl₃): d (ppm) = 19.22 (SiC(CH₃)₃), 26.87 (SiC(CH₃)₃), 27.96 (OC(CH₃)₃), 38.16 (C-4), 39.71 (C-3), 64.14 (C-5), 66.51 (C-6), 83.21 (OC(CH₃)₃), 127.76 (Ar), 127.91 (Ar), 129.19 (Ar), 130.0 (Ar), 132.55 (Ar), 133.0 (Ar), 135.54 (Ar), 142.44 (Ar), 149.59 (C=O Urethan), 173.54 (C=O Lactam)
[α]_{D}²⁰ = -26,5 (c=0,226/CHCl₃)

| | | | | | | |
|---|---|---|---|---|---|---|
| C₃₂H₃₈ClNO₄Si 564,19 | | | | | | |
| ber. | C | 68,12% | H | 6,79% | N | 2,48% |
| gef. | C | 67,90% | H | 6,97% | N | 2,40% |

### Stufe 1-F: 4S,5S-4-Allyl-1-tert.butoxycarbonyl-5-tert.butyldiphenylsilyloxymethylpyrrolidin-2-on (IIIf)

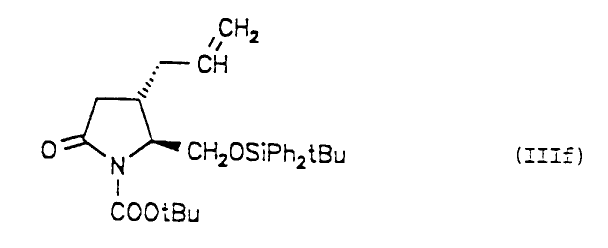

In einem ausgeheizten Schlenk werden unter N₂ 0,52 g (2,5 mmol) CuBrSMe₂ in 5 ml Et₂O suspendiert und bei -30°C 5 ml (5 mmol) Allylmagnesiumbromid 1 M in Et₂O zugegeben. Nach 15 min Rühren kühlt man auf -78°C und tropft 225 mg IIa mit 0,1 ml (lmmol) in 3 ml THF zu. Der Ansatz wird 1,5 h gerührt und mit gesättigter NH₄Cl-Lösung abgestoppt. Die Aufarbeitung erfolgt wie unter IIIa beschrieben.
Ausbeute: 150 mg (60%) farbloses Öl, (R_{f}=0,3 FM: Et₂O/PE 2+3)
IR (Film): 3040, 2940, 2860, 1790, 1750, 1715, 1650, 1590, 1470, 1430, 1370, 1310, 1150, 1110, 790, 760, 700 cm⁻¹ ¹H-NMR (CDCl₃): d (ppm) = 1.03 (9 H, s, Si-t-Bu), 1.42 (9 H, s, O-t-Bu), 2.17 (3 H, m, H-3 und CH₂-Allyl), 2.36 (1 H, quin, J=7,3 Hz, H-4), 2.91 (1 H, dd, J₃₋₄=8,6 Hz, J_{gem}=17,7 Hz, H-3), 3.67 (1 H, dd, J₆ₐ₋₅=4 Hz, J_{gem}=11,9 Hz, H-6), 3.86 (2 H, m, H-5 und H-6), 5.05 (2 H, m, =CH₂), 5.70 (1 H, m, -CH=), 7.37 (6 H, m, H-Ar), 7.59 (4 H, m, H-Ar)
¹³C-NMR (CDCl₃): d (ppm) = 19.16 (SiC(CH₃)₃), 26.80 (SiC(CH₃)₃), 27.99 (OC(CH₃)₃), 32.78 (C-4), 38.11 (C-3), 39.38 (CH₂-Allyl), 63.72 (C-5), 64.61 (C-6), 82.72 (OC(CH₃)₃), 117.88 (=CH-), 127.81 (C-Ar), 129.86 (C-Ar), 132.68 (C-Ar), 113.05 (C-Ar), 134.71 (C-Ar), 135.51 (=CH₂), 149.90 (Urethan), 174.10 (Lactam)
[α]_{D}²⁰ = -32,7 (c=0,22/CHCl3)

| | | | | | | |
|---|---|---|---|---|---|---|
| C₂₉H₃₉NO₄Si 439,72 | | | | | | |
| ber. | C | 70,55% | H | 7,96% | N | 2,84% |
| gef. | C | 70,91% | H | 8,27% | N | 2,60% |

### Stufe 2-E: 4R,5S-1-tert.Butoxycarbonyl-4-(4-chlorphenyl)-5-hydroxymethylpyrrolidin-2-on (IVe)

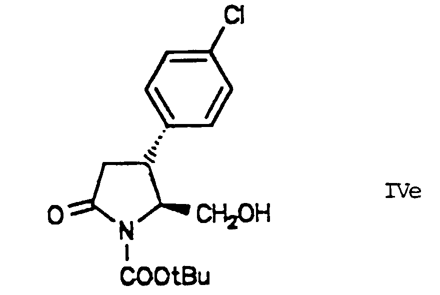

1,12 g (2mmol) IIIe werden in 30 ml THF gelöst und mit 960 mg (8mmnol) Et₃NHF versetzt. Der Ansatz wird 4-5 d bei RT gerührt, mit 30 ml EtOAc verdünnt und mit gesättigter NH₄Cl-Lösung ausgeschüttelt, bis sich kein Niederschlag mehr in der wässrigen Phase bildet. Die vereingten wässrigen Phasen werden mit 2 mal 10 ml EtOAc extrahiert und die organischen Phasen nach Trocknen über Na₂SO₄ im Vakuum eingeengt. Zur Entfernung des Silanols wird über Kieselgel mit EtOAc/PE 2+1 chromatographiert. Nach Einrotieren verbleibt ein farbloses Öl, das bald kristallisiert. Das Produkt kann aus EtOAc/n-Hexan umkristallisiert werden, für die weitere Verarbeitung wurde aber auf eine Umkristallisation verzichtet.
Ausbeute: 400mg (62%) farblose Kristalle (R_{f} = 0,37 FM: EtOAc/PE 2+1)
Smp.: 137°C (EtOAc/n-Hexan)
IR (KBr): 3400 (O-H), 2980-2940, 1790 (C=O), 1500, 1370, 1310, 1170, 1080, 1015, 770 cm⁻¹
¹H-NMR (CDCl₃): d (ppm) = 1.53 (9 H, s, t-Bu), 2.53 (1 H, dd, J₃ₐ₋₄=3,91 Hz, J_{gem}=17,8 Hz, H-3), 2.93 (1 H, t, J =5,31 Hz, O-H), 3.14 (1 H, dd, J_{3b-4}= 9,33 Hz, J_{gem}=17,8 Hz, H-3), 3.43 (1 H, dt, J₃ₐ₋₄J₄₋₅=3,7 Hz, J_{3b-4}=9,2 Hz, H-4), 3.80 (1 H, m, H-6), 3.96 (1 H, m, H-6), 4.08 (1 H, q, J=3,4 Hz, H-5), 7.15 (2 H, d, J_{AB}=8,5 Hz, H-Ar), 7.31 (2 H, d, J_{AB}=8,5 Hz, H-Ar)
¹³C-NMR (CDCl₃): d (ppm) = 27.99 (C(CH₃)₃), 38.07(C-4), 39.72 (C-3), 63.41 (C-5), 66.91 (C-6), 83.79 (C(CH₃)₃), 127.95 (C-Ar), 129.21 (C-Ar), 133.08 (C-Ar), 141.54 (C-Ar), 150.35 (Urethan), 173.73 (Lactam)
[α]_{D}²⁰ = - 43 (c=0,073/CHCl₃)

| | | | | | | |
|---|---|---|---|---|---|---|
| C₁₆H₂₀ClNO₄ 325,79 | | | | | | |
| ber. | C | 58,98% | H | 6,19% | N | 4,30% |
| gef. | C | 58,69% | H | 6,02% | N | 4,30% |

### Stufe 3-E: 2S,3R-1-tert.Butoxycarbonyl-3-(4-chlorphenyl)-5-oxopyrrolidin-2-carbonsäure (Ve)

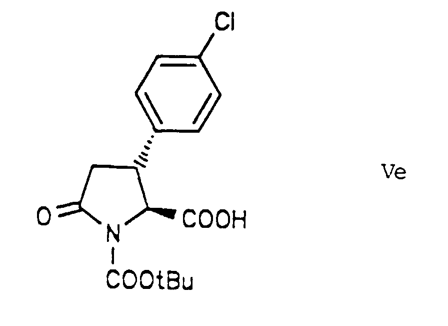

600 mg IVe (1,85 mmol) werden in 8 ml Acetonitril und 4 ml CCl₄ gelöst, dazu gibt man 6 ml H₂O und 1,1 g (5,2 mmol) NaIO₄. Wenn alles gelöst ist, werden 9 mg (0,045 mmol) RuCl₃H₂O zugegeben und der Ansatz bei RT 1-1,5 h stark gerührt. Danach wird mit 15 ml CH₂Cl₂ verdünnt und etwas NH₄Cl-Lösung und 0,5 M HCl zugegeben. Die Wasserphase wird mit CH₂Cl₂ dreimal extrahiert. Die vereinigten organischen Phasen werden mit Na₂SO₄ getrocknet und nach Filtration mit halbgesättigter NaHCO₃-Lösung 3-4 mal extrahiert. Dabei sollten keine schwarzen Flocken in die Wasserphase gelangen. Die Wasserphase wird vorsichtig mit 2 N HCl auf pH 1-2 angesäuert und 3-4 mal mit CH₂Cl₂ extrahiert. Die organischen Phasen werden über Na₂SO₄ getrocknet und das Lösungsmittel abgezogen. Man erhält weiße Kristalle, die aus CHCl₃/PE umkristallisiert werden (450 mg). Die Mutterlauge wird eingeengt und gleichartig behandelt und liefert noch 50 mg Kristalle.
Ausbeute: 500 mg (80%) weiße, feine Kristalle (R_{f} =0,15 FM: EtOAc/CH₂Cl₂/HAC 8+2+1)
Smp.: 141°C (CHCl₃/PE)
IR (KBr): 3200 (breit, O-H), 2970, 1795 (C=O), 1770 (C=O), 1730 (C=O), 1490 (Ar), 1310, 1250, 1155, 830 cm⁻¹
¹H-NMR (CDCl₃): d (ppm) = 1.49 (9 H, s, t-Bu), 2.68 (1 H, dd, J₄ₐ₋₃=4,5 Hz, J_{gem}=17,8 Hz, H-4), 3.09 (1 H, dd, J_{4b-3}=9,2 Hz, J_{gem}=17,8 Hz, H-4), 3.54 (1H, m, H-3), 4.56 (1 H, d, J=3,75 Hz) 7.20 (2 H, d, J_{AB}=8,5 Hz, H-Ar), 7.36 (2 H, d, J_{AB}=8,5 Hz, H-Ar), 9.44 (1 H, s, COOH)
¹³C-NMR (CDCl₃): d (ppm) = 27.84 (C(CH₃)₃), 38.77 (C-3), 39.05 (C-4), 65.89 (C-2), 84.59 (C(CH₃)₃), 127.92 (C-Ar), 129.48 (C-Ar), 133.99 (C-Ar), 139.24 (C-Ar), 149.15 (Urethan), 172.10 (COOH), 175.29 (Lactam)
[α]_{D}²⁰ = +33,7 (c=0,162/CHCl₃)

| | | | | | | |
|---|---|---|---|---|---|---|
| C₁₆H₁₈NO₅Cl 339,78 | | | | | | |
| ber. | C | 56,39% | H | 5,32% | N | 4,11% |
| gef. | C | 56,69% | H | 5,44% | N | 4,24% |

### Stufe 4-E: 4R-1-tert.Butoxycarbonyl-4-(4-chlorphenyl)pyr rolidin-2-on (VIe)

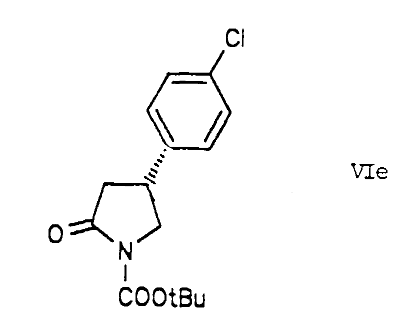

340 mg (lmmol) Ve werden in einem ausgeheizten Schlenk unter N₂ in 9 ml absolutem THF gelöst und bei -15°C mit 102 mg (1mmol) N-Methyl-Morpholin und 136 mg (1mmol) Isobutylchloroformat versetzt. Nach 5 min gibt man 152 mg (1,2 mmol) N-Hydroxy-2-thiopyridon und 122 mg (1,2 mmol) Triethylamin in 4 ml THF zu und läßt bei -15°C 1 h unter Lichtausschluß rühren. Das entstandene Hydrochlorid wird unter Vermeidung von Lichteinwirkung über eine Umkehrfritte abgesaugt, mit THF nachgewaschen und das Filtrat mit 0,9 ml (10 mmol) tert-Butylmercaptan versetzt. Diese Lösung wird mit zwei 100 W Lampen unter Inertgas im Wasserbad bei RT 1 h bestrahlt. Dabei verschwindet die gelbe Farbe weitgehend. Nach 1 h wird mit Ether verdünnt und mit halbgesättigter NaHCO₃-Lösung, Wasser, 0,5 N HCl, Wasser und gesättigter NaCl-Lösung gewaschen. Man tocknet über Na₂SO₄ und zieht das Lösungsmittel ab. Der Rückstand wird mittels SC an SiO₂ mit EtOAc/PE 1+2 gereinigt. Man erhält ein farbloses Öl, das alsbald kristallisiert. Nach Umkristallisieren mit Diisopropylether/PE erhält man farblose, feine Nadeln.
Ausbeute: 190 mg (64%) farblose Nadeln (R_{f} = 0,5 FM: EtOAc/PE 1+2)
Smp.: (Iso₂O/PE): 103°C
IR (KBr): 2990, 1780 (C=O), 1500, 1370, 1290, 1150, 1090, 1015, 840, 780 cm⁻¹
¹H-NMR (CDCl₃): d (ppm) = 1.54 (9 H, s, t-Bu), 2.66 (1 H, dd, J₃ₐ₋₄=9,5 Hz, J_{gem}=17,3 Hz, H-3), 2.90 (1 H, dd, J_{3b-4}=8,2 Hz, J_{gem}=17,3 Hz, H-3), 3.51 (1 H, quin, J₃ₐ₋₄J_{3b-4}J₅ₐ₋₄J_{5b-4}=8,3 Hz, H-4), 3.65 (1 H, dd, J₅ₐ₋₄=8,2 Hz, J_{gem}=10,7 Hz, H-5), 4.15 (1 H, dd, J_{5b-4}=7,5 Hz, J_{gem}=10,5 Hz, H-5), 7.17 (2 H, dt, J=1,9 Hz, J_{AB}=8,4 Hz, H-Ar), 7.33 (2 H, dt, J=2 Hz, J_{AB}=8,5 Hz, H-Ar)
¹³C-NMR (CDCl₃): d (ppm) = 28.01 (OC(CH₃)₃), 35.83 (C-4), 40.19 (C-3), 52.87 (C-5), 83.17 (OC(CH₃)₃), 128.08 (C-Ar), 129.13 (C-Ar), 133.27 (C-Ar), 139.14 (C-Ar), 149.82 (Urethan), 172.41 (Lactam)
[α]_{D}²⁰ = +4,5 (c=0,2/CHCl₃)

| | | | | | | |
|---|---|---|---|---|---|---|
| C₁₅H₁₈NO₃Cl 295,76 | | | | | | |
| ber. | C | 60,92% | H | 6,13% | N | 4,74% |
| gef. | C | 60,94% | H | 6,31% | N | 4,80% |

### Stufe 5-E:3R-4-tert.-Butoxycarbonylamino-3-(4-chlorphenyl)butansäure (VIIe)

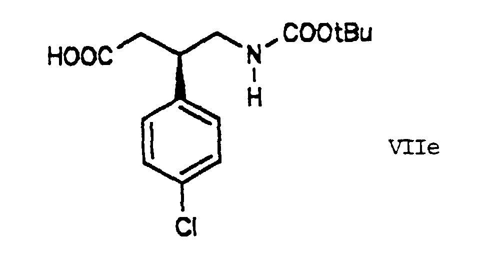

100 mg VIe werden in 3 ml THF gelöst und mit 1 ml 1 M LiOH-Lösung versetzt. Die Mischung wird 1,5 h gerührt, das THF im Vakuum entfernt und die Wasserphase mit 0,5 M HCl auf pH 1-2 gebracht. Man extrahiert dreimal mit 10 ml Et₂O, trocknet mit Na₂SO₄ und zieht den Ether am Rotavapor ab. Man erhält weiße Kristalle, evtl. muß mit Ether angerieben werden. Diese werden aus CHCl₃/PE umkristallisiert. Ausbeute: 90 mg (87%) weiße Blättchen (R_{f}=0,65 FM: EtO-Ac/CH₂Cl₂/HAc 2+8+1)
Smp: 135°-138°C (CHCl₃/PE)
IR (KBr): 3390 (N-H), 3000-2900 und 2630 (COOH), 1690 (C=O), 1530, 1500, 1410, 1370, 1250, 1180, 1020, 830 cm⁻¹ ¹H-NMR (CDCl₃/d₆-DMSO): d (ppm) = 1.39 (9 H, s, t-Bu), 2.50 (1 H, dd, J₂ₐ₋₃=8,4 Hz, J_{gem}=15,9 Hz, H-2), 2.69 (1 H, dd, J_{2b-3}=4,3 Hz, J_{gem} = 16,1 Hz, H-2), 3.28 (3 H, m, H-3 und H-4), 5.87 (1 H, s breit, N-H), 7.19 (2 H, d, J_{AB}=8,66 Hz, H-Ar), 7.26 (2 H, d, J_{AB}=8,67 Hz, H-Ar)
¹³C-NMR (CDCl₃/d₆₋DMSO): d (ppm) = 28.29 (C(CH₃)₃), 37.93 (C-3), 41.59 (C-2), 45.44 (C-4), 78.46 (C(CH₃)₃), 128.28 (C-Ar), 129.29 (C-Ar), 131.83 (C-Ar), 140.78 (C-Ar), 155.95 (Urethan), 173.44 (COOH)
[α]_{D}²⁰ = +14 (c=0.234/MeOH)

| | | | | | | |
|---|---|---|---|---|---|---|
| C₁₅H₂₀NO₄Cl 313,7 | | | | | | |
| ber. | C | 57,42% | H | 6,42% | N | 4,46% |
| gef. | C | 57,24% | H | 6,43% | N | 4,32% |

### Stufe 6-E: 3R-4-Amino-3-(4-chlorphenyl)-butansäure-hydrochlorid (Ie)

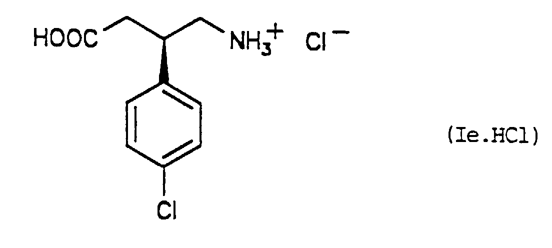

104 mg (0,33 mmol) VIIe werden in 10 ml 6 M HCl suspendiert und 3 h zum Sieden erhitzt. Dabei ergibt sich eine klare Lösung. Die verdünnte HCl wird am Rotavopor abdestillert , der kristalline Rückstand im Exsiccator getrocknet und mit EtOH und Aceton verrieben. Nach 30 min wird das kristalline Hydrochlorid abgesaugt. Man erhält farblose Kristalle.
Ausbeute: 62 mg (74%) farblose Kristalle
Smp.: 219°C
IR (KBr): 3000 (NH₃⁺, COOH), 1720 (COOH), 1580 (NH₃⁺), 1490, 1410, 1400, 1200, 1180, 1125, 1010, 950, 825 cm⁻¹
¹H-NMR (D₂O/d₄-Methanol): d (ppm) = 2.63 (1 H, dd, J₂ₐ₋₃=8,7 Hz, J_{gem}=16,4 Hz, H-2), 2.78 (1 H, dd, J_{2b-3}=5,6 Hz, J_{gem}=16,3 Hz, H-2), 3.24 (3 H, m, H-3 und H-4), 7.25 (2 H, d, J_{AB}=8,5 Hz, H-Ar), 7.34 (2 H, d, J_{AB}=8,55 Hz, H-Ar)
¹³C-NMR: (D₂O/d₄-Methanol): d (ppm) =39.10 (C-3), 40.41 (C-2), 44.62 (C-4), 130.19 (C-Ar), 130.37 (C-Ar), 134.38 (C-Ar), 138.16 (C-Ar), 175.67 (COOH)
[α]_{D}²⁰ = -2 (c=0,2/H₂O) Lit.: -1,5 (c=0,2/H₂O)

| | | | | | | |
|---|---|---|---|---|---|---|
| C₁₀H₁₃NO₂Cl₂ 250,12 | | | | | | |
| ber. | C | 48,02% | H | 5,24% | N | 5,60% |
| gef. | C | 47,65% | H | 5,19% | N | 5,44% |

## Patentansprüche

1. Verfahren zur Herstellung von enantiomerenreinen β-substituierten γ-Aminobuttersäurederivaten der Formel (I) 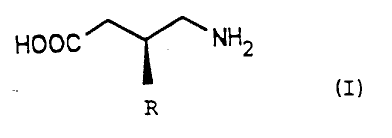 in der R für eine geradkettige oder verzweigtkettige Alkylgruppe mit 1 bis 6 C-Atomen, eine Alkenylgruppe mit 2 bis 6 C-Atomen oder einen gegebenenfalls substituierten Phenylrest steht, der als Substituenten einen oder zwei Substituenten aufweisen kann, die ausgewählt sind aus den Halogenatomen F, Cl, Br, I, Alkylresten mit 1 bis 4 C-Atomen und Alkoxy- oder Cycloalkoxyresten mit 1 bis 6 C-Atomen,
dadurch gekennzeichnet, daß man ein S-Pyroglutaminsäurederivat der allgemeinen Formel (II) 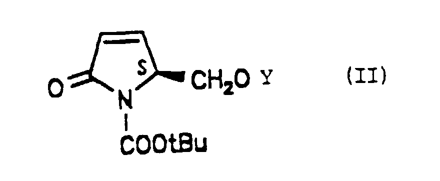 in der Y für eine an sich bekannte sterisch anspruchsvolle OH-Schutzgruppe steht,
mit einem metallorganischen Reagens, das die Einführung eines Restes R selektiv in die der Gruppe -CH₂OY benachbarte 4-Position der Verbindung der allgemeinen Formel (II) ermöglicht, zu einer Verbindung der allgemeinen Formel (III) umsetzt 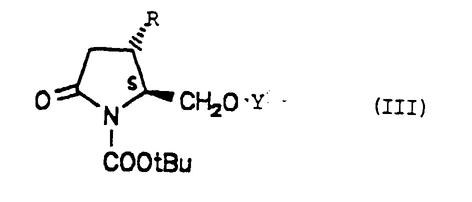 und aus dieser durch an sich bekannte schonende Abspaltung der OH-Schutzgruppe, Oxidation der dabei gebildeten freien Methylolgruppe zu einer Carboxylgruppe und Bildung einer Verbindung der allgemeinen Formel (V), 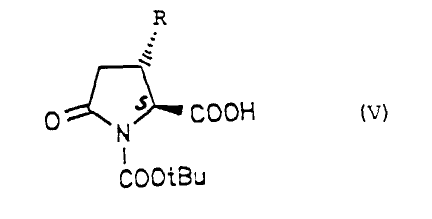 anschließende Decarboxylierung dieser Verbindung der allgemeinen Formel (V) und anschließende hydrolytische Ringöffnung der dabei gebildeten, in 4-Position substituierten γ-Lactamringverbindung der allgemeinen Formel (VI) 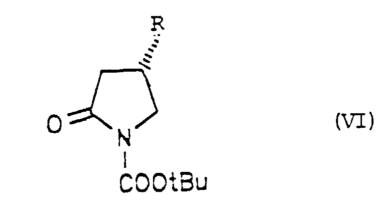 vor oder nach der Abspaltung der tert.-Butoxycarbonylgruppe die Verbindung der allgemeinen Formel (I) in Form eines Säureadditionssalzes oder in freier Form gewinnt.

2. Verfahren nach Anspruch 1, dadurch gekennzeichnet, daß die OH-Schutzgruppe Y ausgewählt ist aus tert.-Butyldiphenylsilyl-, tert.-Butyldimethylsilyl- und Tritylresten.

3. Verfahren nach Anspruch 1 oder 2, dadurch gekennzeichnet, daß die metallorganischen Reagenzien zur Einführung des Restes R aus Cu-organische und Zn-organische Verbindungen enthaltenden Reagenzien ausgewählt sind.

4. Verfahren nach Anspruch 3, dadurch gekennzeichnet, daß das Cu- oder Zn-organische Reagens ausgewählt ist aus Reagenzien, die eine Verbindung mit einer der Formeln R₂CuMgX, in der X für eines der Halogenatome Cl, Br, I steht, R₂CuLi, R₂Zn oder R₂CuZnCl, in denen R jeweils wie bei Formel (I) definiert ist, umfassen.

5. Verfahren nach einem der Ansprüche 1 bis 4, dadurch gekennzeichnet, daß R jeweils für einen p-Cl-Phenylrest steht und die erhaltene Verbindung der Formel (I) R-Baclofen (Ie) ist.

6. Verfahren nach einem der Ansprüche 1 bis 5, dadurch gekennzeichnet, daß man die Oxidation der Methylolgruppe der nach Abspaltung der Schutzgruppe aus der Verbindung der Formel (III) erhaltenen Verbindung mit NaIO₄ in Gegenwart von RuCl₃ oder mit gasförmigem Sauerstoff in Anwesenheit eines Platinmetallkatalysators durchführt.

7. Verfahren nach Anspruch 6, dadurch gekennzeichnet, daß man als Platinmetallkatalysator PtO₂ verwendet.

8. Verfahren nach einem der Ansprüche 1 bis 7, dadurch gekennzeichnet, daß man die Decarboxylierung der Verbindung der Formel (V) nach ihrer Umsetzung mit Isobutylchloroformat und N-Hydroxy-2-thiopyridon photolytisch in Gegenwart von tert.-Butylmercaptan durchführt (Barton Reaktion).

9. Verfahren nach einem der Ansprüche 1 bis 8, dadurch gekennzeichnet, daß man die hydrolytische Ringöffnung der Verbindung der Formel (VI) dadurch durchführt, daß man diese Verbindung mit LiOH umsetzt und danach zur Gewinnung der Verbindung der Formel (I) die tert.-Butoxycarbonylgruppe abspaltet.

10. Ein enantiomerenreines Pyroglutaminsäurederivat der allgemeinen Formel (III) 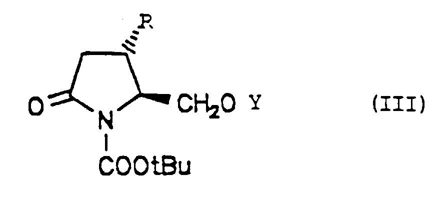 in der R für eine geradkettige oder verzweigtkettige Alkylgruppe mit 1 bis 6 C-Atomen, eine Alkenylgruppe mit 2 bis 6 C-Atomen oder einen gegebenenfalls substituierten Phenylrest steht, der als Substituenten einen oder zwei Substituenten aufweisen kann, die ausgewählt sind aus den Halogenatomen F, Cl, Br, I, Alkylresten mit 1 bis 4 C-Atomen und Alkoxy- oder Cycloalkoxyresten mit 1 bis 6 C-Atomen steht und Y für eine OH-Schutzgruppe steht, die ausgewählt ist aus tert.-Butyldiphenylsilyl-, tert.-Butyldimethylsilyl- und Tritylresten.

11. Ein enantiomerenreines Pyroglutaminsäurederivat der allgemeinen Formel (V) 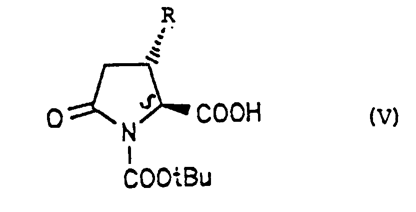 in der R für eine geradkettige oder verzweigtkettige Alkylgruppe mit 1 bis 6 C-Atomen, eine Alkenylgruppe mit 2 bis 6 C-Atomen oder einen gegebenenfalls substituierten Phenylrest steht, der als Substituenten einen oder zwei Substituenten aufweisen kann, die ausgewählt sind aus den Halogenatomen F, Cl, Br, I, Alkylresten mit 1 bis 4 C-Atomen und Alkoxy- oder Cycloalkoxyresten mit 1 bis 6 C-Atomen steht.

12. Verwendung einer Verbindung der allgemeinen Formel (V) gemäß Anspruch 11 zur Herstellung enantiomerenreiner γ-Aminobuttersäurederivat der Formel (I).

## Claims

1. Process for manufacturing enantiomerically pure, β-substituted γ-aminobutyric acid derivatives of Formula (I) 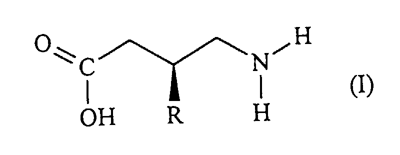 in which R represents a straight-chain or branched-chain alkyl group with 1 to 6 C atoms, an alkenyl group with 2 to 6 C atoms or an optionally substituted phenyl radical that can have as substituents one or two substituents chosen from the halogen atoms F, Cl, Br, I, alkyl radicals with 1 to 4 C atoms and alkoxy or cycloalkoxy radicals with 1 to 6 C atoms,
characterized in that an S-pyroglutamic acid derivative of the general formula (II) 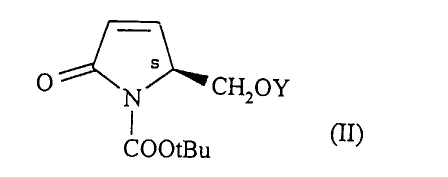 in which Y represents a sterically discriminating OH protection group, which is itself known, is reacted with an organometallic reagent that enables a radical R to be introduced selectively into the 4-position adjacent to the -CH₂OY group of the compound with the general formula (II) to yield a compound of the general formula (III) 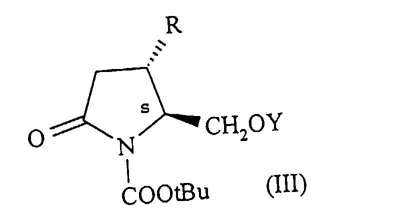 and from this the compound of the general formula (I) is obtained in the form of an acid addition salt or in free form by a known gentle abscission of the OH protective group, oxidizing the free methylol group that is formed thereby to a carboxyl group and formation of a compound of the general formula (V), 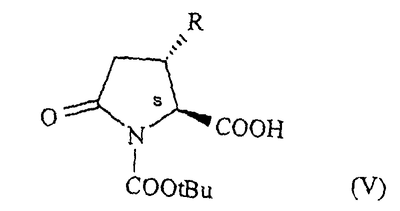 followed by decarboxylation of this compound of general formula (V) and subsequent hydrolytic ring-opening of the γ-lactam ring compound of general formula (VI) substituted in the 4 position that is formed thereby 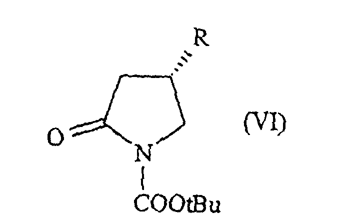 before or after cleaving off the tert-butoxycarbonyl group.

2. Process according to Claim 1, characterized in that the OH protection group Y is selected from tert-butyldiphenylsilyl, tert-butyldimethylsilyl and trityl radicals.

3. Process according to Claim 1 or 2, characterized in that the organometallic reagents to introduce the R radical are selected from reagents containing Cu-organic and Zn-organic compounds.

4. Process according to Claim 3, characterized in that the Cu-organic or Zn-organic reagent is chosen from reagents comprising a compound with one of the formulae R2CuMgX, in which X represents one of the halogen atoms Cl, Br, I, R₂CuLi, R₂Zn or R₂CuZnCl, in each of which R is defined as in Formula (I).

5. Process according to one of the Claims 1 to 4, characterized in that in each case R represents a p-Cl-phenyl radical and the compound of Formula (I) that is obtained is R-Baclofen (Ie).

6. Process according to one of the Claims 1 to 5, characterized in that the oxidation of the methylol group of the compound obtained after splitting off the protective group from the compound of Formula (III) is carried out using NaIO₄ in the presence of RuCl₃ or with gaseous oxygen in the presence of a platinum metal catalyst.

7. Process according to Claim 6, characterized in that PtO₂ is used as the platinum metal catalyst.

8. Process according to one of the Claims 1 to 7, characterized in that the decarboxylation of the compound of Formula (V), after it has reacted with isobutyl chloroformate and N-hydroxy-2-thiopyridone, is carried out photolytically in the presence of tert-butyl mercaptan (Barton Reaction).

9. Process according to one of the Claims 1 to 8, characterized in that the hydrolytic ring-opening of the compound of Formula (VI) is carried out by reacting this compound with LiOH and afterwards splitting off the tert-butoxycarbonyl group to obtain the compound of Formula (I).

10. An enantiomerically pure pyroglutamic acid derivative of the general formula (III) 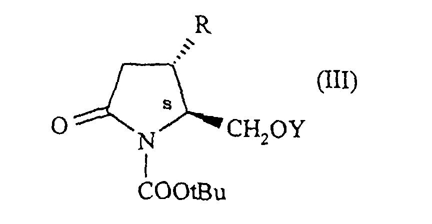 in which R represents a straight-chain or branched-chain alkyl group with 1 to 6 C atoms, an alkenyl group with 2 to 6 C atoms or an optionally substituted phenyl radical that can have as substituents one or two substituents chosen from the halogen atoms F, Cl, Br, I, alkyl radicals with 1 to 4 C atoms and alkoxy or cycloalkoxy radicals with 1 to 6 C atoms, and Y represents an OH protection group selected from tert-butyldiphenylsilyl, tert-butyldimethylsilyl and trityl radicals.

11. An enantiomerically pure pyroglutamic acid derivative of the general formula (V) 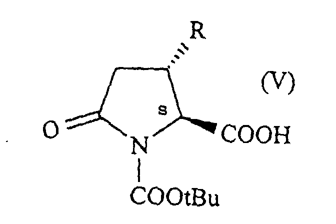 in which R represents a straight-chain or branched-chain alkyl group with 1 to 6 C atoms, an alkenyl group with 2 to 6 C atoms or an optionally substituted phenyl radical that can have as substituents one or two substituents chosen from the halogen atoms F, Cl, Br, I, alkyl radicals with 1 to 4 C atoms and alkoxy or cycloalkoxy radicals with 1 to 6 C atoms.

12. Use of a compound of the general Formula (V) according to Claim 11 to manufacture enantiomerically pure γ-aminobutyric acid derivatives of Formula (I).

## Revendications

1. Procédé pour la préparation de dérivés énantiomériquement purs d'acide γ-aminobutyrique substitué en β de formule (I) 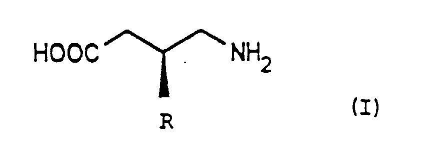 dans laquelle R représente un groupe alkyle à chaîne linéaire ou ramifiée comptant de 1 à 6 atomes de C, un groupe alcényle comptant de 2 à 6 atomes de C ou un résidu phényle éventuellement substitué, qui peut présenter comme substituants un ou deux substituants qui sont choisis parmi les atomes d'halogène F, Cl, Br, I, des résidus alkyle comptant de 1 à 4 atomes de C et des résidus alcoxy ou cycloalcoxy comptant de 1 à 6 atomes de C,
caractérisé en ce que l'on transforme un dérivé d'acide S-pyroglutamique de formule générale (II) 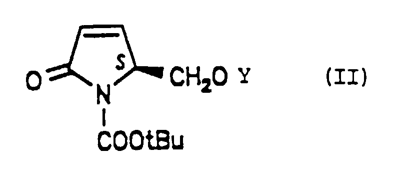 dans laquelle Y représente un groupe de protection OH stériquement gênant connu en soi, avec un réactif organométallique qui permet l'introduction d'un résidu R sélectivement dans la position 4, voisine du groupe -CH₂OY, du composé de formule générale (II), en un composé de formule générale (III) 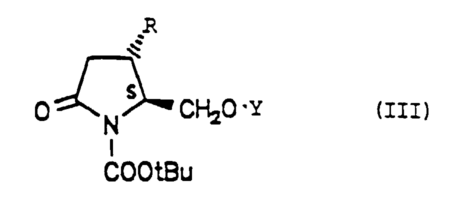 et en ce que, à partir de ce dernier, par une séparation conduite de manière soigneuse, connue en soi, du groupe de protection OH, oxydation du groupe méthylol libre ainsi formé en un groupe carboxyle et formation d'un composé de formule générale (V), 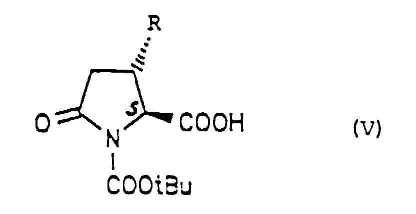 et ensuite par décarboxylation de ce composé de formule générale (V), suivie d'une ouverture de cycle par hydrolyse du composé cyclique ainsi formé, de γ-lactame substitué en position 4, de formule générale (VI) 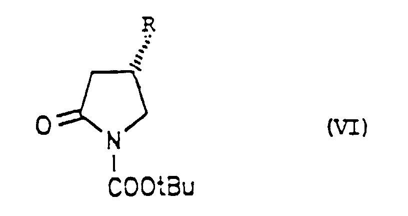 avant ou après la séparation du groupe tert-butoxycarbonyle, on obtient le composé de formule générale (I) sous la forme d'un sel d'addition d'acide ou sous forme libre.

2. Procédé selon la revendication 1, caractérisé en ce que le groupe Y de protection OH est choisi parmi des résidus de tert-butyldiphénylsilyle, de tert-butyldiméthylsilyle et de trityle.

3. Procédé selon la revendication 1 ou 2, caractérisé en ce que les réactifs organométalliques servant à l'introduction du résidu R sont sélectionnés parmi des réactifs contenant des composés organiques de Cu et des composés organiques de Zn.

4. Procédé selon la revendication 3, caractérisé en ce que le réactif organique de Cu ou de Zn est sélectionné parmi des réactifs qui comportent un composé présentant l'une des formules R₂CuMgX, dans laquelle X représente un des atomes d'halogène Cl, Br, I, R₂CuLi, R₂Zn ou R₂CuZnCl, dans lesquelles R est chaque fois défini comme en formule (I).

5. Procédé selon l'une des revendications 1 à 4, caractérisé en ce que R représente chaque fois un résidu de p-Cl-phényle, et le composé de formule (I) ainsi obtenu est le R-baclofen (Ie).

6. Procédé selon l'une des revendications 1 à 5, caractérisé en ce que l'on effectue l'oxydation du groupe méthylol du composé obtenu après séparation du groupe de protection du composé de formule (III) par NaIO₄ en présence de RuCl₃, ou par l'oxygène gazeux en présence d'un catalyseur du groupe du platine.

7. Procédé selon la revendication 6, caractérisé en ce que l'on utilise PtO₂ comme catalyseur du groupe du platine.

8. Procédé selon l'une des revendications 1 à 7, caractérisé en ce que l'on effectue la décarboxylation du composé de formule (V) après sa réaction avec le chloroformate d'isobutyle et la N-hydroxy-2-thiopyridone, par photolyse en présence de tert-butylmercaptan (réaction de Barton).

9. Procédé selon l'une des revendications 1 à 8, caractérisé en ce que l'on effectue l'ouverture du cycle par hydrolyse du composé de formule (VI) en faisant réagir ce composé avec LiOH, et qu'ensuite on sépare le groupe tert-butoxycarbonyle en vue d'obtenir le composé de formule (I).

10. Dérivé d'acide pyroglutamique énantiomériquement pur de formule générale (III) 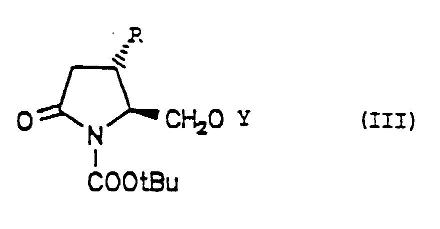 dans laquelle R représente un groupe alkyle à chaîne linéaire ou ramifiée comptant de 1 à 6 atomes de C, un groupe alcényle comptant de 2 à 6 atomes de C ou un résidu phényle éventuellement substitué qui peut présenter comme substituants un ou deux substituants qui sont sélectionnés parmi les atomes d'halogène F, Cl, Br, I, des résidus alkyle comptant de 1 à 4 atomes de C et des résidus alcoxy ou cycloalcoxy comptant de 1 à 6 atomes de C, et Y représente un groupe de protection OH qui est choisi parmi des résidus de tert-butyldiphénylsilyle, de tert-butyldiméthylsilyle et de trityle.

11. Dérivé énantiomériquement pur d'acide pyroglutamique de formule générale (V) 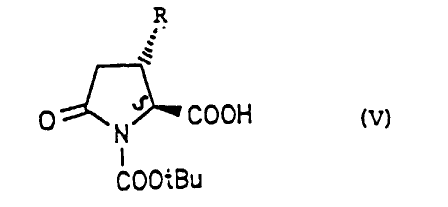 dans laquelle R représente un groupe alkyle à chaîne linéaire ou ramifiée comptant de 1 à 6 atomes de C, un groupe alcényle comptant de 2 à 6 atomes de C ou un résidu phényle éventuellement substitué qui peut présenter comme substituants un ou deux substituants qui sont sélectionnés parmi les atomes d'halogène F, Cl, Br, I, des résidus alkyle comptant de 1 à 4 atomes de C et des résidus alcoxy ou cycloalcoxy comptant de 1 à 6 atomes de C.

12. Utilisation d'un composé de formule générale (V) selon la revendication 11 pour la préparation d'un dérivé énantiomériquement pur d'acide γ-aminobutyrique de formule (I).
